# FASCICULE DE BREVET EUROPEEN

(11) **EP 0 894 795 B1**
(45) Date de publication et mention de la délivrance du brevet: **06.06.2001**
(21) Numéro de dépôt: 98401920.8
(22) Date de dépôt: 28.07.1998
(51) Int. Cl.: C07D 403/04, A61K 31/415, C07D 417/04, A61K 31/425, C07D 471/04, C07D 401/04, C07D 413/04, A61K 31/42, C07D 409/04, C07D 405/04

(54) **Nouveaux dérivés de benzimidazole, de benzoxazole et de benzothiazole, leur procédé de préparation et les compositions pharmaceutiques qui les contiennent**
Benzimidazol-, Benzoxazol-, und Benzothiazolderivate, Verfahren zu ihrer Herstellung und pharmazeutische Zusammensetzungen die sie enthalten
Benzimidazole, benzoxazole and benzothiazole derivatives, their preparation and pharmaceutical compositions containing them

(30) Priorité: 30.07.1997 FR 9709710
(43) Date de publication de la demande: 03.02.1999
(73) Titulaire: ADIR ET COMPAGNIE, 92415 Courbevoie Cédex (FR)
(72) Inventeur: De Nanteuil, Guillaume, 92150 Suresnes (FR); Portevin, Bernard, 78990 Elancourt (FR); Bonnet, Jacqueline, 75013 Paris (FR); Fradin, Armel, 92200 Neuilly Sur Seine (FR)

(56) Documents cités:
- EP-A- 0 820 990
- WO-A-96/11917
- US-A- 5 612 360

## Description

La présente invention concerne de nouveaux dérivés de benzimidazole, de benzoxazole et de benzothiazole, leur procédé de préparation et les compositions pharmaceutiques qui les contiennent. Ces composés, outre le fait qu'ils soient nouveaux, sont de puissants inhibiteurs de l'interleukine 1β (IL1β) et de sa formation.

L'IL1β est produite par les macrophages et possède une grande variété d'activités biologiques reliées à des pathologies inflammatoires comme l'arthrite rhumatoïde ou l'arthrose.

L'IL1β stimule les cellules présentes dans l'articulation qui synthétisent et expriment alors la cyclooxygénase inductible (COX2), ainsi que la NO synthase inductible, pour fournir les prostaglandines et le NO, qui sont des médiateurs importants de la douleur et de l'inflammation. L'IL1β active également l'expression et la synthèse de protéases qui participent à la dégradation de la matrice extracellulaire des chondrocytes et à la suppression de la synthèse des composants de la matrice du cartilage. De plus, l'IL1β intervient dans l'activation des cellules endothéliales qui expriment alors différents facteurs d'adhésion, ainsi que dans l'induction d'autres cytokines pro-inflammatoires comme le TNF ou les chémokines (l'IL6). Enfin, l'IL1β joue un rôle dans la régulation de la résorption osseuse ainsi que dans la différentiation et la prolifération lymphocytaire. On peut donc attendre d'un inhibiteur de l'IL1β qu'il agisse contre les phénomènes inflammatoires et modifie de façon favorable l'evolution de pathologies comme l'arthrite rhumatoïde ou l'arthrose.

L'état de la technique est notamment représenté par le brevet EP-A-820 990.

Plus spécifiquement, la présente invention concerne les composés de formule (I) : 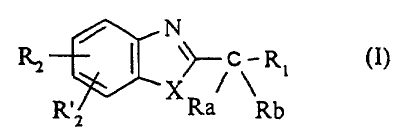 dans laquelle :
- R₁: représente un atome d'halogène, un groupement hydroxy, alkoxy (C₁-C₆) linéaire ou ramifié (éventuellement substitué par un groupement aryle), trihalogénométhyle, arylméthyle, cyano, sulfo, amino (éventuellement substitué par un ou plusieurs groupements, identiques ou différents, alkyle (C₁-C₆) linéaire ou ramifié, aryle éventuellement substitué, acyle, arylsulfonyle ou alkylsulfonyle), mercapto, alkylthio (C₁-C₆) linéaire ou ramifié, trialkylammonium (C₁-C₆) linéaire ou ramifié, aryloxy, arylthio, arylaminocarbonyle, arylcarbonylamino, arylsulfonylamino, arylaminosulfonyle, arylureïdo, arylthioureïdo, arylsulfonyle, arylsulfonyloxy, cycloalkyl (C₃-C₇) oxy, cycloalkyl (C₃-C₇) thio, bicycloalkyl (C₆-C₈) oxy (substitué éventuellement par un groupement aryle), bicycloalkyl (C₆-C₈) thio (éventuellement substitué par un groupement aryle), étant entendu que chaque groupement aryle peut être éventuellement substitué,
- Ra, Rb,: identiques ou différents, représentent un atome d'hydrogène, un groupement hydroxy ou un groupement alkyle (C₁-C₆) linéaire ou ramifié (éventuellement substitué par un groupement aryle),
- X: représente un atome d'oxygène ou de soufre ou un groupement NR (dans lequel R représente un atome d'hydrogène ou un groupement alkyle (C₁-C₆) linéaire ou ramifié),
- R₂: représente un groupement imidazolyle, benzimidazol-1-yle, naphtyle, pyridyle, thiazolyle, oxazolyle, benzothiazolyle, benzoxazolyle, pyrrolyle, furyle, thiényle, imidazopyridinyle, triazolyle ou tétrazolyle, chacun de ces groupements étant éventuellement substitué par un ou plusieurs, identiques ou différents, atomes d'halogène, groupements alkyle (C₁-C₆) linéaire ou ramifié, trihalogénoalkyle (C₁-C₆) linéaire ou ramifié, alkoxy (C₁-C₆) linéaire ou ramifié, nitro, cyano, carboxy, alkoxycarbonyle (C₁-C₆) linéaire ou ramifié, hydroxy, amino (substitué éventuellement par un ou plusieurs groupements, identiques ou différents, alkyle (C₁-C₆) linéaire ou ramifié, acyle, alkylsulfonyle ou arylsulfonyle), phényle éventuellement substitué ou bicycloalkyle éventuellement substitué,
- R'₂: représente un atome d'hydrogène ou groupement aryle éventuellement substitué,
leurs stéréoisomères ainsi que leurs sels d'addition à un acide ou à une base pharmaceutiquement acceptable.

Par groupement aryle, on entend un groupement aromatique, mono ou bicyclique, contenant éventuellement 1 à 4 hétéroatomes choisis parmi azote, soufre ou oxygène. Le terme éventuellement substitué s'appliquant aux groupements aryle, phényle ou bicycloalkyle signifie une substitution de ces groupements par un ou plusieurs, identiques ou différents, atomes d'halogène ou groupements alkyle (C₁-C₆) linéaire ou ramifié, trihalogénoalkyle (C₁-C₆) linéaire ou ramifié, alkoxy (C₁-C₆) linéaire ou ramifié (éventuellement substitué par un groupement carboxy ou alkoxycarbonyle), hydroxy, nitro, cyano, amino (substitué éventuellement par un ou plusieurs groupements, identiques ou différents, alkyle (C₁-C₆) linéaire ou ramifié, acyle, alkylsulfonyle ou arylsulfonyle), carboxy, alkoxycarbonyle (C₁-C₆) linéaire ou ramifié, hydroxyaminocarbonyle, alkylsulfonylamino, arylsulfonylamino, alkylsulfonylaminocarbonyle, arylsulfonylaminocarbonyle, phényle éventuellement substitué ou bicycloalkyle éventuellement substitué.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) dans lesquels X représente un groupement NR tel que défini précédemment.

Le groupement R₂ préféré est le groupement imidazolyle éventuellement substitué.

Les groupements R₁ préférés selon l'invention sont les groupements aryloxy éventuellement substitués, arylthio éventuellement substitués ou arylsulfonylamino éventuellement substitué.

La présente invention s'étend également au procédé de préparation des composés de formule (I). Lorsque les composés de formule (I) que l'on souhaite obtenir sont tels que X = NR, le procédé est caractérisé en ce que l'on utilise comme produit de départ un composé de formule (II) : 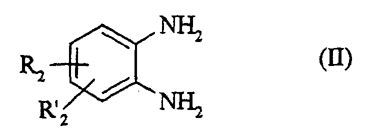 dans laquelle R₂ et R'₂ sont tels que définis dans la formule (I),
que l'on fait réagir, en milieu acide, avec un composé de formule (III) : 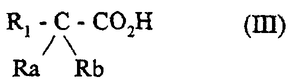 dans laquelle Rₐ, R_{b} et R₁ ont la même signification que dans la formule (I),
pour conduire au composé de formule (I/a), cas particulier des composés de formule (I) : 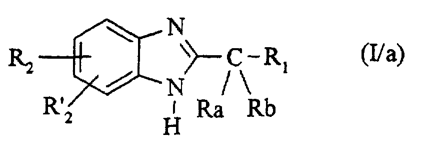 dans laquelle R₁, Rₐ, R_{b}, R₂ et R'₂ sont tels que définis dans la formule (I),
qui subit, éventuellement lorsque R₁ représente un groupement hydroxy, l'action du chlorure de thionyle,
pour conduire au composé de formule (I/b), cas particulier des composés de formule (I) 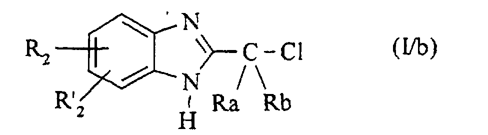 dans laquelle R₂, R'₂, Rₐ et R_{b} ont la même signification que dans la formule (I),
qui peut alors subir les réactions classiques que l'on peut mettre en oeuvre sur des dérivés chlorés, pour obtenir les substitutions correspondantes,
composé de formule (I/a) ou (I/b) :
- dont la fonction NH peut être éventuellement substituée par un groupement alkyle (C₁-C₆) linéaire ou ramifié,
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

Lorsque les composés de formule (I) que l'on souhaite obtenir sont tels que X= X' = O ou S, le procédé est caractérisé en ce que l'on utilise comme produit de départ un composé de formule (IV) : 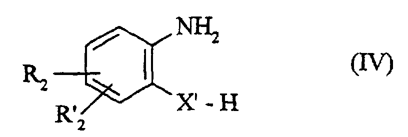 dans laquelle R₂ et R'₂ sont tels que définis dans la formule (I) et X' = O ou S,
que l'on fait réagir, en milieu acide, avec un composé de formule (III) : 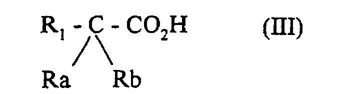 dans laquelle Rₐ, R_{b} et R₁ ont la même signification que dans la formule (I),
pour conduire au composé de formule (I/c), cas particulier des composés de formule (I) : 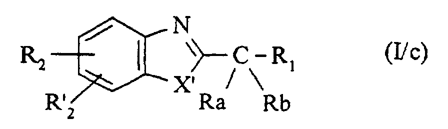 dans laquelle R₂, R'₂, X', Rₐ, R_{b} et R sont tels que définis précédemment,
qui subit, éventuellement lorsque R₁ représente un groupement hydroxy, l'action du chlorure de thionyle,
pour conduire au composé de formule (I/d), cas particulier des composés de formule (I) : 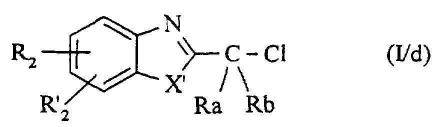 dans laquelle R₂, R'₂, Rₐ, R_{b} et X' ont la même signification que précédemment,
qui peut alors subir les réactions classiques que l'on peut mettre en oeuvre sur des dérivés chlorés, pour obtenir les substitutions correspondantes,
composé de formule (I/c) ou (I/d) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

Les composés de formules (II), (III) ou (IV) sont, quant à eux, obtenus selon des méthodes classiques de la littérature adaptées à la nature et à la position des substituants R₂ et R'₂ sur le noyau phényle pour les composés de formule (II) ou (IV) ou Rₐ et R_{b} pour les composés de formule (III).

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, patentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,1 à 100 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples et les préparations ont été déterminées selon les techniques spectrophotométriques usuelles (infrarouge, RMN, spectrométrie de masse, ...).

### EXEMPLE 1 : 2-Phénoxyméthyl-5-(imidazol-1-yl)benzimidazole, dichlorhydrate

### Stade A : 1-(3-Amino-4-nitrophényl)imidazole

Dans un ballon de un litre, sont agitées 240 mmoles d'imidazole, 240 mmoles de 2-amino-4-chloronitrobenzène et 240 mmoles de carbonate de potassium dans 450 ml de diméthylformamide (DMF). L'ensemble est maintenu 48 heures à 130°C sous agitation. Après évaporation du DMF, le résidu est repris par de l'eau. Le précipité qui se forme est filtré, puis lavé à l'eau. Il est ensuite dissous dans 550 ml d'acide chlorhydrique IN. Après filtration de l'insoluble, le filtrat est alcalinisé et le précipité formé est filtré et lavé à l'eau. Après séchage, le produit attendu est obtenu après purification par chromatographie sur gel de silice en utilisant un mélange dichlorométhane/méthanol (95/5).
***Point de fusion : 189°C***

### Stade B : 1-(3,4-Diaminophényl)imidazole

93 mmoles du composé obtenu au stade précédent sont hydrogénées, à pression atmosphérique, dans 400 ml d'un mélange éthanol/dioxane (50/50) en présence d'1 g de catalyseur Pd/C. Après 18 heures d'hydrogénation, le milieu est filtré, le filtrat évaporé et on obtient le produit attendu.
***Point de fusion: 172°C***

### Stade C : 2-Phénoxyméthyl-5-(imidazol-1-yl)benzimidazole, dichlorhydrate

34,4 mmoles du produit obtenu au stade précédent et 51 mmoles d'acide phénoxyacétique sont portées 18 h à reflux dans 120 ml d'acide chlorhydrique 4N. Après refroidissement, le milieu est alcalinisé à l'aide de carbonate de potassium. Le précipité qui se forme est dissous dans du méthanol. La solution méthanolique est décolorée sur charbon puis évaporée. Le résidu est alors purifié par chromatographie sur gel de silice en utilisant comme éluant un mélange dichlorométhane/méthanol/ammoniaque (95/5/0,5). Le produit purifié est alors transformé en dichlorhydrate correspondant par dissolution dans de l'acide chlorhydrique 1N, évaporation et séchage.
***Point de fusion : 186°C***

| *Microanalyse élémentaire :* | | | | |
|---|---|---|---|---|
| | *C%* | *H%* | *N%* | *Cl%* |
| *calculé* | *56*,*21* | *4,44* | *15*,*42* | *19,52* |
| *trouvé* | *56*,*60* | *4*,*96* | *15*,*50* | *19*,*36* |

Les produits des exemples suivants ont été obtenus selon le procédé décrit dans l'exemple 1, en utilisant les produits de départ correspondants :

### EXEMPLE 2 : 2-Hydroxyméthyl-5-(imidazolyl-1-yl)benzimidazole, dichlorhydrate

***Point de fusion : 235°C***

| *Microanalyse élémentaire :* | | | | |
|---|---|---|---|---|
| | *C%* | *H%* | *N%* | *CI%* |
| *calculé* | *46,01* | *4,21* | *19,51* | *24,69* |
| *trouvé* | *45,64* | *4,24* | *19,04* | *24,94* |

### EXEMPLE 3 : 2-(4-Méthoxyphénoxy)méthyl-5-(imidazol-1-yl)benzimidazole, dichlorhydrate

***Point de fusion : 190-192°C (déc.)***

| *Microanalyse élémentaire :* | | | | |
|---|---|---|---|---|
| | *C %* | *H %* | *N %* | *Cl %* |
| *calculé* | *54,97* | *4,61* | *14,25* | *18,03* |
| *trouvé* | *54,66* | *5,10* | *14,02* | *18,03* |

### EXEMPLE 4 : 2-(4-Fluorophénoxy)méthyl-5-(imidazol-1-yl)benzimidazole, dichlorhydrate

***Point de fusion : 216-218°C***

| *Microanalyse élémentaire :* | | | | |
|---|---|---|---|---|
| | *C %* | *H %* | *N %* | *Cl %* |
| *calculé* | *53,56* | *3,97* | *14,70* | *18,60* |
| *trouvé* | *53,47* | *4,41* | *14,57* | *18,67* |

### EXEMPLE 5 : 2-(2,4-Dichlorophénoxy)méthyl-5-(imidazol-1-yl)benzimidazole, dichlorhydrate

***Point de fusion : 255-260°C***

| *Microanalyse élémentaire :* | | | | |
|---|---|---|---|---|
| | *C %* | *H %* | *N %* | *Cl %* |
| *calculé* | *47,25* | *3,27* | *12,97* | *32,82* |
| *trouvé* | *46,56* | *3,69* | *12,54* | *32,71* |

### EXEMPLE 6 : 2-(4-Chlorophénoxy)méthyl-5-(imidazol-1-yl)benzimidazole, dichlorhydrate

***Point de fusion : 255°C***

| *Microanalyse élémentaire :* | | | | |
|---|---|---|---|---|
| | *C %* | *H %* | *N %* | *Cl* |
| *calculé* | *51,34* | *3,80* | *14,09* | *26,74* |
| *trouvé* | *51,91* | *3,74* | *14,05* | *26,23* |

### EXEMPLE 7 : 2-Phénylthiométhyl-5-(imidazol-1-yl)benzimidazole, dichlorhydrate

***Point de fusion : 189°C***

| *Microanalyse élémentaire :* | | | | | |
|---|---|---|---|---|---|
| | *C%* | *H%* | *N%* | *CI%* | *S%* |
| *calculé* | *53*,*83* | *4*,*25* | *14*,*77* | *18*,*69* | *8*,*45* |
| *trouvé* | *54*,*07* | *4*,*23* | *14*,*60* | *19*,*19* | *8*,*36* |

### EXEMPLE 8 : 2-Phénylsulfonylméthyl-5-(imidazol-1-yl)benzimidazole

| *Microanalyse élémentaire :* | | | | | |
|---|---|---|---|---|---|
| | *C%* | *H%* | *N%* | *CI%* | *S%* |
| *calculé* | *49*,*64* | *3*,*92* | *13*,*62* | *17*,*24* | *7*,*80* |
| *trouvé* | *49*,*95* | *3*,*83* | *13*,*38* | *17*,*62* | *7*,*76* |

### EXEMPLE 9 : 2-Hydroxyméthyl-5-(benzimidazol-1-yl)benzimidazole, dichlorhydrate

***Point de fusion : > 260°C***

| *Microanalyse élémentaire :* | | | | |
|---|---|---|---|---|
| | *C %* | *H %* | *N %* | *Cl %* |
| *calculé* | *53,43* | *4,18* | *16,61* | *21,03* |
| *trouvé* | *53,34* | *3,99* | *16,35* | *21,30* |

### EXEMPLE 10 : 2-Phénoxyméthyl-5-(benzimidazol-1-yl)benzimidazole, dichlorhydrate

***Point de fusion : 210°C (déc.)***

| *Microanalyse élémentaire :* | | | | |
|---|---|---|---|---|
| | *C %* | *H %* | *N %* | *Cl %* |
| *calculé* | *61,03* | *4,39* | *13,56* | *17,16* |
| *trouvé* | *60,77* | *4,48* | *13,13* | *17,01* |

### EXEMPLE 11 : 2-Phénoxyméthyl-5-(4-phénylimidazol-1-yl)benzimidazole

***Point de fusion : 240°C***

| *Microanalyse élémentaire :* | | | |
|---|---|---|---|
| | *C %* | *H %* | *N %* |
| *calculé* | *75,39* | *4,95* | *15,29* |
| *trouvé* | *74,59* | *5,03* | *15,20* |

### EXEMPLE 12 : 2-Hydroxyméthyl-5-(4-phénylimidazol-1-yl)benzimidazole, dichlorhydrate

***Point de fusion : > 260°C***

| *Microanalyse élémentaire :* | | | | |
|---|---|---|---|---|
| | *C %* | *H %* | *N %* | *Cl %* |
| *calculé* | *56,21* | *4,44* | *15,42* | *19,52* |
| *trouvé* | *56,21* | *4,37* | *15,44* | *19,35* |

### EXEMPLE 13 : 2-Phénoxyméthyl-5-(1,2,4-triazol-4-yl)benzimidazole, dichlorhydrate

***Point de fusion : 220°C***

| *Microanalyse élémentaire :* | | | | |
|---|---|---|---|---|
| | *C %* | *H %* | *N %* | *Cl %* |
| *calculé* | *52,76* | *4,15* | *19,29* | *19,47* |
| *trouvé* | *52,54* | *4,67* | *19,05* | *21,09* |

### EXEMPLE 14 : 2-Phénoxyméthyl-5-(pyrrol-1-yl)benzimidazole

***Point de fusion : 120-122°C***

| *Microanalyse élémentaire :* | | | |
|---|---|---|---|
| | *C %* | *H %* | *N %* |
| *Calculé* | *74,72* | *5,23* | *14,52* |
| *trouvé* | *74,65* | *5,52* | *14.02* |

### EXEMPLE 15 : 2-(3-Trifluorophénylthiométhyl)-5-(imidazol-1-yl)benzimidazole

### EXEMPLE 16 : 2-Phénoxyméthyl-5-(benzothiazol-2-yl)benzimidazole, chlorhydrate

### Stade A : 2-(3,4-Diaminophényl)benzothiazole

Le produit attendu est obtenu selon le procédé décrit au stade A de l'exemple 15 à partir du 2-(3,4-dinitrophényl)benzothiazole.

### Stade B : 2-Phénoxyméthyl-5-(benzothiazol-2-yl)benzimidazole, chlorhydrate

Le produit attendu est obtenu selon le procédé décrit au stade C de l'exemple 1 à partir du composé obtenu au stade précédent.
***Point de fusion : 222°C***

| *Microanalyse élémentaire :* | | | | | |
|---|---|---|---|---|---|
| | *C %* | *H %* | *N %* | *Cl %* | *S %* |
| *calculé* | *64,03* | *4,10* | *10,67* | *9,00* | *8,14* |
| *trouvé* | *63,60* | *4,19* | *10,56* | *9,43* | *8,06* |

### EXEMPLE 17 : 2-Phénoxyméthyl-5-(imidazol[1,2-a]pyridin-2-yl)benzimidazole

Le produit attendu est obtenu selon le procédé décrit dans l'exemple 16 en utilisant les produits de départ correspondants.

| *Microanalyse élémentaire :* | | | |
|---|---|---|---|
| | *C %* | *H %* | *N %* |
| *calculé* | *74,10* | *4,74* | *16,46* |
| *trouvé* | *73,92* | *4,69* | *16,27* |

### EXEMPLE 18 : 2-Phénoxyméthyl-5-(2-méthylthiazol-4-yl)benzimidazole

Le produit attendu est obtenu selon le procédé décrit dans l'exemple 16 en utilisant les produits de départ correspondants.

| *Microanalyse élémentaire :* | | | | |
|---|---|---|---|---|
| | *C %* | *H %* | *N %* | *S %* |
| *calculé* | *67,27* | *4,70* | *13,07* | *9,98* |
| *trouvé* | *67,64* | *4,62* | *13,01* | *10,16* |

Les exemples suivants ont été préparés selon le procédé décrit dans l'exemple 1 en utilisant les produits de départ correspondants.

### EXEMPLE 19 : 2-Cyclohexyloxyméthyl-5-(imidazol-1-yl)benzimidazole

| *Microanalyse élémentaire :* | | | |
|---|---|---|---|
| | *C %* | *H %* | *N %* |
| *calculé* | *68,90* | *6,80* | *18,90* |
| *trouvé* | *69,02* | *6,70* | *18,99* |

### EXEMPLE 20 : 2-Biphényloxyméthyl-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *62,88* | *4,59* | *12,75* | *16,14* |
| *trouvé* | *62,95* | *4,88* | *12,81* | *16,49* |

### EXEMPLE 21 : 2-(3,5-Ditrifluorométhyl)phénoxyméthyl-5-(imidazol-1-yl) benzimidazole

| *Microanalyse élémentaire :* | | | |
|---|---|---|---|
| | *C %* | *H %* | *N %* |
| *calculé* | *53,53* | *2,84* | *13,14* |
| *trouvé* | *53,65* | *3,04* | *13,48* |

### EXEMPLE 22 : 2-(3,4-Diméthoxyphénylthiométhyl-5-(imidazol-1-yl)benzimidazole, dichlorhydrate

| *Microanalyse élémentaire :* | | | | | |
|---|---|---|---|---|---|
| | *C %* | *H %* | *N %* | *Cl %* | *S %* |
| *calculé* | *51,94* | *4,59* | *12,75* | *16,14* | *7,30* |
| *trouvé* | *51,44* | *4,65* | *12,33* | *16,45* | *7,22* |

### EXEMPLE 23 : 2-(4-Méthoxyphénylthiométhyl)-5-(imidazol-1-yl)benzimidazole

### EXEMPLE 24 : 2-(3-Trifluorométhylphénoxyméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *50,13* | *3,51* | *12,99* | *16,44* |
| *trouvé* | *50,92* | *3,67* | *13,12* | *15,81* |

### EXEMPLE 25 : 2-(2,6-Diméthylphénoxyméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *58,32* | *5,15* | *14,32* | *18,12* |
| *trouvé* | *58,30* | *5,27* | *14,09* | *18,23* |

### EXEMPLE 26 : 2-(3,4,5-Triméthoxyphénoxyméthyl)-5-(imidazol-1-yl)-benzimidazole, dichlorhydrate

| *Microanalyse élémentaire :* | | | | |
|---|---|---|---|---|
| | *C %* | *H %* | *N %* | *Cl %* |
| *calculé* | *52,99* | *4,89* | *12,36* | *15,64* |
| *trouvé* | *53,15* | *5,41* | *12,17* | *14,50* |

### EXEMPLE 27 : 2-(Phénylaminométhyl)-5-(imidazol-1-yl)benzimidazole

| *Microanalyse élémentaire :* | | | |
|---|---|---|---|
| | *C %* | *H %* | *N %* |
| *calculé* | *70,57* | *5,23* | *24,20* |
| *trouvé* | *70,54* | *5,20* | *24,03* |

### EXEMPLE 28 : 2-[(2,6-Ditertbutyl-4-hydroxy)phénylthiométhyl])-5-(imidazol-1-yl) benzimidazole

### EXEMPLE 29 : 2-(1-(S)-Phénoxyéthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *57,31* | *4,81* | *14,85* | *18,79* |
| *trouvé* | *57,30* | *4,89* | *14,98* | *19,21* |

### EXEMPLE 30 : 2-(1-(R)-Phénoxyéthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *57,31* | *4,81* | *14,85* | *18,79* |
| *trouvé* | *57,31* | *4,84* | *14,82* | *19,09* |

### EXEMPLE 31 : 2-(N-Méthylanilinométhyl)- 5-(imidazol-1-yl)benzimidazole

| *Microanalyse élémentaire :* | | | |
|---|---|---|---|
| | *C %* | *H %* | *N %* |
| *calculé* | *71,27* | *5,65* | *23,09* |
| *trouvé* | *70,80* | *5,83* | *22,74* |

### EXEMPLE 32 : 2-(2-Méthoxyphénylthiométhyl)-5-(imidazol-1-yl)benzimidazole, dichlorhydrate

| *Microanalyse élémentaire :* | | | | | |
|---|---|---|---|---|---|
| | *C%* | *H%* | *N%* | *Cl%* | *S%* |
| *calculé* | *52,82* | *4,43* | *13,69* | *17,32* | *7,83* |
| *trouvé* | *52*,*70* | *4*,*58* | *13*,*63* | *17*,*88* | *8*,*00* |

### EXEMPLE 33 : 2-(4-Acétamidophénylthiométhyl)-5-(imidazol-1-yl)benzimidazole, dichlorhydrate

| *Microanalyse élémentaire :* | | | | | |
|---|---|---|---|---|---|
| | *C%* | *H%* | *N%* | *Cl%* | *S%* |
| *calculé* | *52*,*30* | *4*,*39* | *16*,*05* | *16*,*25* | *7*,*35* |
| *trouvé* | *51*,*98* | *4,49* | *15*,*95* | *16,58* | *7,08* |

### EXEMPLE 34 : 2-(2,6-Diméthylphénylthiométhyl)-5-(imidazol-1-yl)benzimidazole, dichlorhydrate

| *Microanalyse élémentaire :* | | | | | |
|---|---|---|---|---|---|
| | *C %* | *H %* | *N %* | *Cl %* | *S %* |
| *calculé* | *56,02* | *4,95* | *13,75* | *17,41* | *7,87* |
| *trouvé* | *56,32* | *5,08* | *13,66* | *16,92* | *7,78* |

### EXEMPLE 35 : 2-(4-Hydroxyphénylthiométhyl)-5-(imidazol-1-yl)benzimidazole

| *Microanalyse élémentaire :* | | | | |
|---|---|---|---|---|
| | *C%* | *H%* | *N%* | *S%* |
| *calculé* | *63*,*34* | *4*,*38* | *17*,*38* | *9*,*95* |
| *trouvé* | *63*,*35* | *4,63* | *17*,*39* | *9*,*72* |

### EXEMPLE 36 : 2-(2,6-Dichlorophénoxyméthyl)-5-(imidazol-1-yl)benzimidazole, dichlorhydrate

| *Microanalyse élémentaire :* | | | | |
|---|---|---|---|---|
| | *C%* | *H%* | *N%* | *CI%* |
| *calculé* | *47*,*25* | *3*,*27* | *12*,*97* | *32*,*82* |
| *trouvé* | *47*,*36* | *3*,*46* | *12*,*74* | *31*,*79* |

### EXEMPLE 37 : 2-(2,6-Diméthoxyphénoxyméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *53,91* | *4,76* | *13,24* | *16,75* |
| *trouvé* | *54,05* | *4,99* | *13,14* | *16,92* |

### EXEMPLE 38 : 2-(2,6-Diisopropylphénoxyméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *61,75* | *6,31* | *12,52* | *15,85* |
| *trouvé* | *62,45* | *6,47* | *12,55* | *14,99* |

### EXEMPLE 39 : 2-(2,4,6-Triméthylphénoxyméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *59,27* | *5,47* | *13,82* | *17,49* |
| *trouvé* | *59,36* | *5,82* | *13,78* | *17,23* |

### EXEMPLE 40 : 2-(Cyanométhyl)-5-(imidazol-1-yl)benzimidazole

| *Microanalyse élémentaire :* | | | |
|---|---|---|---|
| | *C %* | *H %* | *N %* |
| *calculé* | *64,56* | *4,06* | *31,37* |
| *trouvé* | *64,14* | *4,34* | *31,58* |

### EXEMPLE 41 : 2-(1-(S)-Hydroxy-2-phényléthyl)-5-(imidazol-1-yl)benzimidazole, dichlorhydrate

| *Microanalyse élémentaire :* | | | | |
|---|---|---|---|---|
| | *C %* | *H%* | *N%* | *Cl %* |
| *calculé* | *57,31* | *4,81* | *14,85* | *18,79* |
| *trouvé* | *56,84* | *5,52* | *14,55* | *18,76* |

### EXEMPLE 42 : 2-(1-(R)-Hydroxy-2-phényléthyl)-5-(imidazol-1-yl)benzimidazole, dichlorhydrate

| *Microanalyse élémentaire :* | | | | |
|---|---|---|---|---|
| | *C%* | *H%* | *N%* | *CI%* |
| *calculé* | *57*,*31* | *4*,*81* | *14*,*85* | *18*,*79* |
| *trouvé* | *56*,*81* | *5*,*51* | *14*,*65* | *18*,*99* |

### EXEMPLE 43 : 2-(4-Carboxyphénoxyméthyl)-5-(imidazol-1-yl)benzimidazole

| *Microanalyse élémentaire:* | | | |
|---|---|---|---|
| | *C%* | *H%* | *N%* |
| *calculé* | *64,67* | *4,22* | *16,76* |
| *trouvé* | *64,21* | *4,25* | *16,59* |

### EXEMPLE 44 : 2-(2-Ethoxycarbonylphénylthiométhyl)-5-(imidazol-1-yl)benzimidazole

| *Microanalyse élémentaire:* | | | | |
|---|---|---|---|---|
| | *C %* | *H %* | *N %* | *S %* |
| *calculé* | *63,47* | *4,79* | *14,80* | *8,47* |
| *trouvé* | *63,87* | *4,94* | *14,70* | *8,31* |

### EXEMPLE 45 : 2-{4-(Carboxyméthoxy)phénylthiométhyl)-5-(imidazol-1-yl) benzimidazole

| *Microanalyse élémentaire:* | | | | |
|---|---|---|---|---|
| | *C %* | *H %* | *N %* | *S %* |
| *calculé* | *59,99* | *4,24* | *14,73* | *8,43* |
| *trouvé* | *60,22* | *4,29* | *14,76* | *8,67* |

### EXEMPLE 46 : 2-(Anilinocarbonylméthyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *55,40* | *4,39* | *17,94* | *18,17* |
| *trouvé* | *55,02* | *4,66* | *17,69* | *18,28* |

### EXEMPLE 47 : 2-(Benzoylaminométhyl)-5-(imidazol-1-yl)benzimidazole, dichlorhydrate

| *Microanalyse élémentaire :* | | | | |
|---|---|---|---|---|
| | *C %* | *H %* | *N %* | *Cl %* |
| *calculé* | *55,39* | *4,39* | *17,95* | *18,17* |
| *trouvé* | *55,85* | *4,73* | *18,18* | *17,67* |

### EXEMPLE 48 : 2-(Phénylsulfonylaminométhyl)-5-(imidazol-1-yl)benzimidazole, dichlorhydrate

| *Microanalyse élémentaire :* | | | | | |
|---|---|---|---|---|---|
| | *C %* | *H %* | *N %* | *Cl %* | *S %* |
| *calculé* | *47,90* | *4,02* | *16,43* | *16,63* | *7,52* |
| *trouvé* | *48,10* | *4,05* | *16,22* | *16,87* | *7,26* |

### EXEMPLE 49 : 2-(Hydroxyméthyl)-5-(imidazol-1-yl)-6-(4-fluorophényl)benzimidazole, dichlorhydrate

| *Microanalyse élémentaire :* | | | | |
|---|---|---|---|---|
| | *C %* | *H %* | *N %* | *Cl %* |
| *calculé* | *53,56* | *3,97* | *14,70* | *18,60* |
| *trouvé* | *53,28* | *3,77* | *14,68* | *19,24* |

### EXEMPLE 50 : 2-(2,6-Diméthoxyphénoxyméthyl)-5-(imidazol-1-yl)-6-(4-fluorophényl) benzimidazole

| *Microanalyse élémentaire :* | | | |
|---|---|---|---|
| | *C %* | *H %* | *N %* |
| *calculé* | *67,56* | *4,76* | *12,61* |
| *trouvé* | *67,34* | *4,98* | *12,63* |

### EXEMPLE 51 : 2-(2,6-Diméthoxyphénoxyméthyl)-5-(pyridin-4-yl)benzimidazole

### EXEMPLE 52 : 2-(2,6-Diméthoxyphénoxyméthyl)-5-(oxazol-4-yl)benzimidazole

### EXEMPLE 53 : 2-(2,6-Diméthylphénylthiométhyl)-5-(thiazol-4-yl)benzimidazole

### EXEMPLE 54 : 2-(4-Hydroxyaminocarbonylphénoxyméthyl)-5-(imidazol-1-yl) benzimidazole

### EXEMPLE 55 : 2-(4-Hydroxyaminocarbonylphénylureïdométhyl)-5-(imidazol-1-yl) benzimidazole

### EXEMPLE 56 : 2-(4-Méthylsulfonylaminophénoxyméthyl)-5-(imidazol-1-yl) benzimidazole

### EXEMPLE 57 : 2-(Méthylsulfonylaminocarbonylphénoxyméthyl)-5-(imidazol-1-yl) benzimidazole

### EXEMPLE 58 : 2-Sulfométhyl-5-(imidazol-1-yl)benzimidazole

### EXEMPLE 59 : 2-(Phénylaminosulfonylméthyl)-5-(imidazol-1-yl)benzimidazole

### EXEMPLE 60 : 2-(Tétrazol-5-yl)méthyl-5-(imidazol-1-yl)benzimidazole

### EXEMPLE 61 : 2-(3,4-Dicarboxyméthoxyphénoxyméthyl)-5-(imidazol-1-yl) benzimidazole

### EXEMPLE 62 : 2-(2,4,6-Triméthoxyphénoxyméthyl)5-(imidazol-1-yl)benzimidazole

### EXEMPLE 63 : 2-(Pyrrol-2-yloxyméthyl)-5-(imidazol-1-yl)benzimidazole

### EXEMPLE 64 : 2-(2,6-Diéthoxyphénoxyméthyl)-5-(imidazol-1-yl)benzimidazole

### EXEMPLE 65 : 2-(2-Méthoxy-6-méthylphénoxyméthyl)-5-(imidazol-1-yl)benzimidazole

### EXEMPLE 66 : 2-(2,6-Dihydroxyphénoxyméthyl)-5-(imidazol-1-yl)benzimidazole

### EXEMPLE 67 : 2-(2-Méthoxy-6-hydroxyphénoxyméthyl)-5-(imidazol-1-yl) benzimidazole

### EXEMPLE 68 : 2-(2-Hydroxyméthyl-6-méthoxyphénoxyméthyl)-5-(imidazol-1-yl) benzimidazole

### EXEMPLE 69 : 2-(2-Méthoxyméthyl-6-méthoxyphénoxyméthyl)-5-(imidazol-1-yl) benzimidazole

### EXEMPLE 70 : 2-(2,6-Diméthoxyphénylthiométhyl)-5-(imidazol-1-y)benzimidazole

### EXEMPLE 71 : 2-(2,6-Diéthoxyphénylthiométhyl)-5-(imidazol-1-yl)benzimidazole

### EXEMPLE 72 : 2-(2-Méthoxy-6-méthylphénylthiométhyl)-5-(imidazol-1-yl) benzimidazole

### EXEMPLE 73 : 2-(2,6-Dihydroxyphénylthiométhyl)-5-(imidazol-1-yl)benzimidazole

### EXEMPLE 74 : 2-(2-Méthoxy-6-hydroxyphénylthiométhyl)-5-(imidazol-1-yl) benzimidazole

### EXEMPLE 75 : 2-(2,6-Diméthoxyphénylaminométhyl)-5-(imidazol-1-yl)benzimidazole

### EXEMPLE 76 : 2-(2,6-Diéthoxyphénylaminométhyl)-5-(imidazol-1-yl)benzimidazole

### EXEMPLE 77 : 2-(2-Méthoxy-6-méthylaminométhyl)-5-(imidazol-1-yl)benzimidazole

### EXEMPLE 78 : 2-(2,6-Dihydroxyphénylaminométhyl)-5-(imidazol-1-yl)benzimidazole

### EXEMPLE 79 : 2-(2-Méthoxy-6-hydroxyphénylaminométhyl)-5-(imidazol-1-yl) benzimidazole

### EXEMPLE 80 : 2-[N-(2,6-Diméthoxyphényl)-N-méthylaminométhyl]-5-(imidazol-1-yl) benzimidazole

### EXEMPLE 81 : 2-[N-(2,6-Diéthoxyphényl)-N-méthylaminométhyl]-5-(imidazol-1-yl) benzimidazole

### EXEMPLE 82 : 2-[N-(2-Méthoxy-6-méthylphényl)-N-méthylaminométhyl]-5-(imidazol-1-yl)benzimidazole

### EXEMPLE 83 : 2-[N-(2,6-Dihydroxyphényl)-N-méthylaminométhyl]-5-(imidazol-1-yl) benzimidazole

### EXEMPLE 84 : 2-[N-(2-Méthoxy-6-hydroxyphényl)-N-méthylaminométhyl]-5-(imidazol-1-yl)benzimidazole

### EXEMPLE 85 : 2-(2,6-Diméthoxyphénylthiométhyl)-5-(imidazol-1-yl)-6-(4-fluorophényl)benzimidazole

### EXEMPLE 86 : 2-(2,6-Diméthoxyphénylaminométhyl)-5-(imidazol-1-yl)-6-(4-fluorophényl)benzimidazole

### EXEMPLE 87 : 2-(2,6-Diméthoxyphénoxyméthyl)-5-(imidazol-1-yl)-6-(imidazol-1-yl) benzimidazole

### EXEMPLE 88 : 2-(2,6-Diméthoxyphénylthiométhyl)-5-(imidazol-1-yl)-6-(imidazol-1-yl) benzimidazole

### EXEMPLE 89 : 2-(2,6-Diméthoxyphénylaminométhyl)-5-(imidazol-1-yl)-6-(imidazol-1-yl)benzimidazole

### EXEMPLE 90 : 2-(2,6-Diméthoxyphénoxyméthyl)-5-(imidazol-1-yl)-6-(4-pyridyl) benzimidazole

### EXEMPLE 91 : 2-(2,6-Diméthoxyphénylthiométhy)-5-(imidazol-1-yl)-6-(4-pyridyl) benzimidazole

### EXEMPLE 92 : 2-(2,6-Diméthoxyphénylaminométhyl)-5-(imidazol-1-yl)-6-(4-pyridyl) benzimidazole

### EXEMPLE 93 : 2-(2,6-Diméthoxyphénoxyméthyl)-5-(imidazol-1-yl)-6-(4'-fluorobiphényl)benzimidazole

### EXEMPLE 94 : 2-(2,6-Diméthoxyphénylthiométhyl)-5-(imidazol-1-yl)-6-(4'-fluorobiphényl)benzimidazole

### EXEMPLE 95 : 2-(2,6-Diméthoxyphénylaminométhyl)-5-(imidazol-1-yl)-6-(4'-fluorobiphényl)benzimidazole

### EXEMPLE 96 : 2-(2,6-Diméthoxyphénoxyméthyl)-5-(imidazol-1-yl)-6-[4-(4-pyridyl) phényl]benzimidazole

### EXEMPLE 97 : 2-(2,6-Diméthoxyphénylthiométhyl)-5-(imidazol-1-yl)-6-[4-(4-pyridyl) phényl]benzimidazole

### EXEMPLE 98 : 2-(2,6-Diméthoxyphénylaminométhyl)-5-(imidazol-1-yl)-6-[4-(4-pyridyl)phényl]benzimidazole

### EXEMPLE 99 : 2-[N-(2,6-Diméthoxyphényl)-N-méthylaminométhyl]-5-(imidazol-1-yl)-6-(4-fluorophényl)benzimidazole

### EXEMPLE 100 : 2-[N-(2,6-Diméthoxyphényl)-N-méthylaminométhyl]-5-(imidazol-1-yl)-6-(imidazol-1-yl)benzimidazole

### EXEMPLE 101 : 2-[N-(2,6-Diméthoxyphényl)-N-méthylaminométhyl]-5-(imidazol-1-yl)-6-(4-pyridyl)benzimidazole

### EXEMPLE 102 : 2-[N-(2,6-Diméthoxyphényl)-N-méthylaminométhyl]-5-(imidazol-1-yl)-6-(4'-fluorobiphényl)benzimidazole

### EXEMPLE 103 : 2-[N-(2,6-Diméthoxyphényl)-N-méthylaminométhyl]-5-(imidazol-1-yl)-6-[4-(4-pyridyl)phényl]benzimidazole

### Etude pharmacologique des dérivés de l'invention

### EXEMPLE 104 :

Les composés ont été étudiés sur la lignée cellulaire humaine THP1 de type monocyte/macrophage. La production d'IL 1β par ces cellules a été obtenue après stimulation avec du lipopolysaccharide bactérien (*M*. *Turner et coll*., *Biochem. Biophys. Res. Comm*., *1988*, *256(2)*, *830-839)* et dosée par la méthode EIA (kit Cayman) selon les instructions fournies par le fabricant. Sur le test du choc endotoxique provoqué chez la souris par injection intraveineuse de lipopolysaccharide, les composés de l'invention ont diminué les taux circulants de TNFa pour une dose orale inférieure ou égale à 100 mg.kg. Les ED₅₀ ont été de 30, 10, 3 et 10 mg/kg p.o. pour les composés des exemples 48, 34, 37 et 50 respectivement.

### EXEMPLE 105 : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 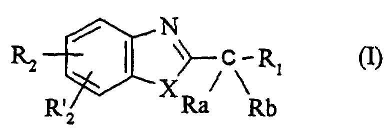 dans laquelle :
R₁ représente un atome d'halogène, un groupement hydroxy, alkoxy (C₁-C₆) linéaire ou ramifié (éventuellement substitué par un groupement aryle), trihalogénométhyle, arylméthyle, cyano, sulfo, amino (éventuellement substitué par un ou plusieurs groupements, identiques ou différents, alkyle (C₁-C₆) linéaire ou ramifié, aryle éventuellement substitué, acyle, arylsulfonyle ou alkylsulfonyle), mercapto, alkylthio (C₁-C₆) linéaire ou ramifié, trialkylammonium (C₁-C₆) linéaire ou ramifié, aryloxy, arylthio, arylaminocarbonyle, arylcarbonylamino, arylsulfonylamino, arylaminosulfonyle, arylureïdo, arylthioureïdo, arylsulfonyle, arylsulfonyloxy, cycloalkyl (C₃-C₇) oxy, cycloalkyl (C₃-C₇) thio, bicycloalkyl (C₆-C₈) oxy (substitué éventuellement par un groupement aryle), bicycloalkyl (C₆-C₈) thio (éventuellement substitué par un groupement aryle), étant entendu que chaque groupement aryle peut être éventuellement substitué,
Ra, Rb, identiques ou différents, représentent un atome d'hydrogène, un groupement hydroxy ou un groupement alkyle (C₁-C₆) linéaire ou ramifié (éventuellement substitué par un groupement aryle),
X représente un atome d'oxygène ou de soufre ou un groupement NR (dans lequel R représente un atome d'hydrogène ou un groupement alkyle (C₁-C₆) linéaire ou ramifié),
R₂ représente un groupement imidazolyle, benzimidazol-1-yle, naphtyle, pyridyle, thiazolyle, oxazolyle, benzothiazolyle, benzoxazolyle, pyrrolyle, furyle, thiényle, imidazopyridinyle, triazolyle ou tétrazolyle, chacun de ces groupements étant éventuellement substitué par un ou plusieurs, identiques ou différents, atomes d'halogène, groupements alkyle (C₁-C₆) linéaire ou ramifié, trihalogénoalkyle (C₁-C₆) linéaire ou ramifié, alkoxy (C₁-C₆) linéaire ou ramifié, nitro, cyano, carboxy, alkoxycarbonyle (C₁-C₆) linéaire ou ramifié, hydroxy, amino (substitué éventuellement par un ou plusieurs groupements, identiques ou différents, alkyle (C₁-C₆) linéaire ou ramifié, acyle, alkylsulfonyle ou arylsulfonyle), phényle éventuellement substitué ou bicycloalkyle éventuellement substitué,
R'₂ représente un atome d'hydrogène ou groupement aryle éventuellement substitué,
leurs stéréoisomères ainsi que leurs sels d'addition à un acide ou à une base pharmaceutiquement acceptable.

2. Composés de formule (I) selon la revendication 1 tels que X représente un groupement NR

3. Composés de formule (I) selon la revendication 1 tels que R₁ représente un groupement aryloxy éventuellement substitué.

4. Composés de formule (I) selon la revendication 1 tels que R₁ représente un groupement arylthio éventuellement substitué.

5. Composés de formule (I) selon la revendication 1 tels que R₁ représente un groupement arylsulfonylamino éventuellement substitué.

6. Composés de formule (I) selon la revendication 2 tel que R₁ représente un groupement phénoxy éventuellement substitué.

7. Composés de formule (I) selon la revendication 4 tel que R₁ représente un groupement phénylthio éventuellement substitué.

8. Composés de formule (I) selon la revendication 5 tel que R₁ représente un groupement phénylsulfonylamino éventuellement substitué.

9. Composés de formule (I) selon la revendication 1 tels que R₂ représente un groupement imidazolyle éventuellement substitué.

10. Composé de formule (I) selon la revendication 1 qui est le 2-phénoxyméthyl-5-(imidazol-l-yl)benzimidazole, ainsi que ses sels d'addition.

11. Composé de formule (I) selon la revendication 1 qui est le 2-phénylthiométhyl-5-(imidazol-1-yl)benzimidazole, ainsi que ses sels d'addition.

12. Composé de formule (I) selon la revendication 1 qui est le 2-(2,6-diméthylphénylthiométhyl)-5-(imidazol-1-yl)benzimidazole, dichlorhydrate.

13. Composé de formule (I) selon la revendication 1 qui est le 2-(2,6-diméthoxyphénoxyméthyl)-5-(imidazol-1-yl)benzimidazole, dichlorhydrate.

14. Composé de formule (I) selon la revendication 1 qui est le 2-(phénylsulfonylaminométhyl)-5-(imidazol-1-yl)benzimidazole, dichlorhydrate.

15. Composé de formule (I) selon la revendication 1 qui est le 2-(2,6-diméthoxyphénoxyméthyl)-5-(imidazol-1-yl)-6-(4-fluorophényl) benzimidazole.

16. Procédé de préparation des composés de formule (I) caractérisé lorsque les composés de formule (I) que l'on souhaite obtenir sont tels que X = NR, le procédé est caractérisé en ce que l'on utilise comme produit de départ un composé de formule (II) : 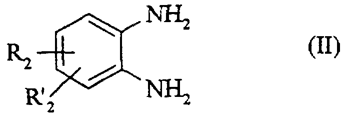 dans laquelle R₂ et R'₂ sont tels que définis dans la formule (I),
que l'on fait réagir, en milieu acide, avec un composé de formule (III) : 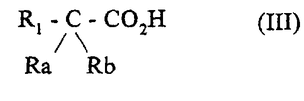 dans laquelle Rₐ, R_{b} et R₁ ont la même signification que dans la formule (I),
pour conduire au composé de formule (I/a), cas particulier des composés de formule (I): 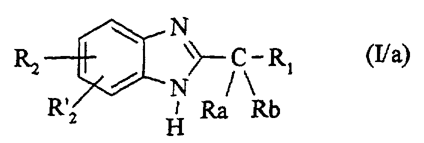 dans laquelle R₁, Rₐ, R_{b}, R₂ et R'₂ sont tels que définis dans la formule (I),
qui subit, éventuellement lorsque R₁ représente un groupement hydroxy, l'action du chlorure de thionyle,
pour conduire au composé de formule (I/b), cas particulier des composés de formule (I): 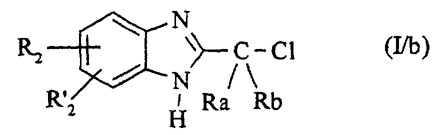 dans laquelle R₂, R'₂, Rₐ et R_{b} ont la même signification que dans la formule (I),
qui peut alors subir les réactions classiques que l'on peut mettre en oeuvre sur des dérivés chlorés, pour obtenir les substitutions correspondantes,
composé de formule (I/a) ou (I/b) :
- dont la fonction NH peut être éventuellement substituée par un groupement alkyle (C₁-C₆) linéaire ou ramifié,
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

17. Procédé de préparation des composés de formule (I) caractérisé lorsque les composés de formule (I) que l'on souhaite obtenir sont tels que X= X' = O ou S, le procédé est caractérisé en ce que l'on utilise comme produit de départ un composé de formule (IV): 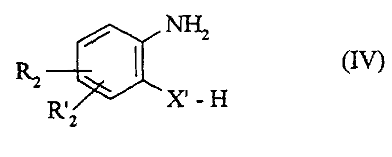 dans laquelle R₂ et R'₂ sont tels que définis dans la formule (I) et X' = O ou S,
que l'on fait réagir, en milieu acide, avec un composé de formule (III) : 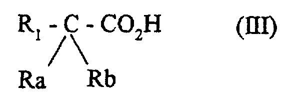 dans laquelle Rₐ, R_{b} et R₁ ont la même signification que dans la formule (I),
pour conduire au composé de formule (I/c), cas particulier des composés de formule (I) : 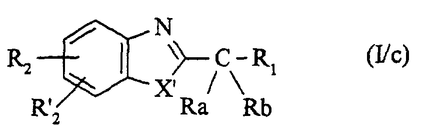 dans laquelle R₂, R'₂, X', Rₐ, R_{b}, et R sont tels que définis précédemment,
qui subit, éventuellement lorsque R₁ représente un groupement hydroxy, l'action du chlorure de thionyle,
pour conduire au composé de formule (I/d), cas particulier des composés de formule (I): 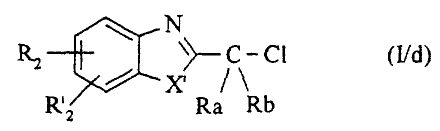 dans laquelle R₂, R'₂, Rₐ et R_{b} ont la même signification que précédemment,
qui peut alors subir les réactions classiques que l'on peut mettre en oeuvre sur des dérivés chlorés, pour obtenir les substitutions correspondantes
composé de formule (I/c) ou (I/d) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

18. Compositions pharmaceutiques contenant comme principe actif au moins un composé selon l'une quelconque des revendications 1 à 15 seul ou en combinaison avec un ou plusieurs excipients ou véhicules inertes, non toxiques, pharmaceutiquement acceptables.

19. Compositions pharmaceutiques selon la revendication 18 contenant au moins un principe actif selon l'une quelconque des revendications 1 à 15 utiles comme inhibiteurs d'interleukine 1β.

## Patentansprüche

1. Verbindungen der Formel (I): 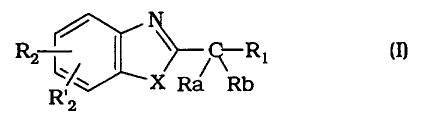 in der:
R₁ ein Wasserstoffatom, eine Hydroxygruppe, eine geradkettige oder verzweigte (gegebenenfalls durch eine Arylgruppe substituierte) (C₁-C₆)-Alkylgruppe, Trihalogenmethylgruppe, Arylmethylgruppe, Cyanogruppe, Sulfogruppe, (gegebenenfalls durch eine oder mehrere gleichartige oder verschiedene geradkettige oder verzweigte (C₁-C₆)-Alkylgruppen, gegebenenfalls substituierte Arylgruppen, Acylgruppen, Arylsulfonylgruppen oder Alkylsulfonylgruppen substituierte) Aminogruppe, Mercaptogruppe, geradkettige oder verzweigte (C₁-C₆)-Alkylthiogruppe, geradkettige oder verzweigte (C₁-C₆)-Trialkylammoniumgruppe, Aryloxygruppe, Arylthiogruppe, Arylaminocarbonylgruppe, Arylcarbonylaminogruppe, Arylsulfonylaminogruppe, Arylaminosulfonylgruppe, Arylureidogruppe, Arylthioureidogruppe, Arylsulfonylgruppe, Arylsulfonyloxygruppe, (C₃-C₇)-Cycloalkyloxygruppe, (C₃-C₇)-Cycloalkylthiogruppe, (gegebenenfalls durch eine Arylgruppe substituierte) (C₆-C₈)-Bicycloalkyloxygruppe, (gegebenenfalls durch eine Arylgruppe substituierte) (C₆-C₈)-Bicycloalkylthiogruppe darstellt, mit der Maßgabe, daß jede Arylgruppe gegebenenfalls substituiert sein kann,
Ra und Rb, die gleichartig oder verschieden sind, Wasserstoffatome, Hydroxygruppen oder geradkettige oder verzweigte (gegebenenfalls durch eine Arylgruppe substituierte) (C₁-C₆)-Alkylgruppen darstellen,
X ein Sauerstoff- oder Schwefelatom oder eine Gruppe NR (in der R ein Wasserstoffatom oder eine geradkettige oder verzweigte (C₁-C₆)-Alkylgruppe darstellt) bedeutet,
R₂ eine Imidazolylgruppe, Benzimidazol-1-yl-gruppe, Naphthylgruppe, Pyridylgruppe, Thiazolylgruppe, Oxazolylgruppe, Benzothiazolylgruppe, Benzoxazolylgruppe, Pyrrolylgruppe, Furylgruppe, Thienylgruppe, Imidazopyridinylgruppe, Triazolylgruppe oder Tetrazolylgruppe, wobei jede dieser Gruppen gegebenenfalls durch ein oder mehrere, gleichartige oder verschiedene Halogenatome, geradkettige oder verzweigte (C₁-C₆)-Alkylgruppen, geradkettige oder verzweigte (C₁-C₆)-Trihalogenalkylgruppen, geradkettige oder verzweigte (C₁-C₆)-Alkoxygruppen, Nitrogruppen, Cyanogruppen, Carboxygruppen, geradkettige oder verzweigte (C₁-C₆)-Alkoxycarbonylgruppen, Hydroxygruppen, (gegebenenfalls durch eine oder mehrere gleichartige oder verschiedene, geradkettige oder verzweigte (C₁-C₆)-Alkylgruppen, Acylgruppen, Alkylsulfonylgruppen oder Arylsulfonylgruppen substituierte) Aminogruppen, gegebenenfalls substituierte Phenylgruppen oder gegebenenfalls substituierte Bicycloalkylgruppen substituiert sein können, bedeutet, und
R'₂ ein Wasserstoffatom oder eine gegebenenfalls substituierte Arylgruppe darstellt,
deren Stereoisomere sowie deren Additionssalze mit einer pharmazeutisch annehmbaren Säure oder Base.

2. Verbindungen der Formel (I) nach Anspruch 1, worin X eine Gruppe NR bedeutet.

3. Verbindungen der Formel (I) nach Anspruch 1, worin R₁ eine gegebenenfalls substituierte Aryloxygruppe darstellt.

4. Verbindungen der Formel (I) nach Anspruch 1, worin R₁ eine gegebenenfalls substituierte Arylthiogruppe darstellt.

5. Verbindungen der Formel (I) nach Anspruch 1, worin R₁ eine gegebenenfalls substituierte Arylsulfonylaminogruppe darstellt.

6. Verbindungen der Formel (I) nach Anspruch 2, worin R₁ eine gegebenenfalls substituierte Phenoxygruppe darstellt.

7. Verbindungen der Formel (I) nach Anspruch 4, worin R₁ eine gegebenenfalls substituierte Phenylthiogruppe darstellt.

8. Verbindungen der Formel (I) nach Anspruch 5, worin R₁ eine gegebenenfalls substituierte Phenylsulfonylaminogruppe darstellt.

9. Verbindungen der Formel (I) nach Anspruch 1, worin R₂ eine gegebenenfalls substituierte Imidazolylgruppe darstellt.

10. Verbindung der Formel (I) nach Anspruch 1, nämlich 2-Phenoxymethyl-5-(imidazol-1-yl)-benzimidazol sowie dessen Additionssalze.

11. Verbindung der Formel (I) nach Anspruch 1, nämlich 2-Phenylthiomethyl-5-(imidazol-1-yl)-benzimidazol sowie dessen Additionssalze.

12. Verbindung der Formel (I) nach Anspruch 1, nämlich 2-(2,6-Dimethylphenylthiomethyl)-5-(imidazol-1-yl)-benzimidazol-Dihydrochlorid.

13. Verbindung der Formel (I) nach Anspruch 1, nämlich 2-(2,6-Dimethoxyphenoxymethyl)-5-(imidazol-1-yl)-benzimidazol-Dihydrochlorid.

14. Verbindung der Formel (I) nach Anspruch 1, nämlich 2-(Phenylsulfonylaminomethyl)-5-(imidazol-1-yl)-benzimidazol-Dihydrochlorid.

15. Verbindung der Formel (I) nach Anspruch 1, nämlich 2-(2,6-Dimethoxyphenoxymethyl)-5-(imidazol-1-yl)-6-(4-fluorphenyl)-benzimidazol.

16. Verfahren zur Herstellung der Verbindungen der Formel (I), dadurch gekennzeichnet, daß, wenn die herzustellenden Verbindungen der Formel (I) jene sind, worin X = NR bedeutet, das Verfahren dadurch gekennzeichnet ist, daß man als Ausgangsprodukt eine Verbindung der Formel (II) verwendet: 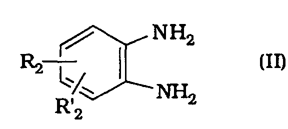 in der R₂ und R'₂ die bezüglich der Formel (I) angegebenen Bedeutungen besitzen, welche man in saurem Medium mit einer Verbindung der Formel (III) umsetzt: 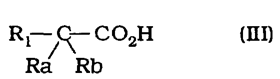 in der Rₐ, R_{b} und R₁ die bezüglich der Formel (I) angegebenen Bedeutungen besitzen,
zur Bildung der Verbindung der Formel (I/a), einem Sonderfall der Verbindungen der Formel (I): 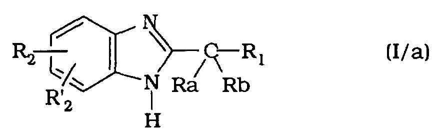 in der R₁, Rₐ, R_{b}, R₂ und R'₂ die bezüglich der Formel (I) angegebenen Bedeutungen besitzen,
welche man gegebenenfalls, wenn R₁ eine Hydroxygruppe darstellt, der Einwirkung von Thionylchlorid unterwirft,
zur Bildung der Verbindung der Formel (I/b), einem Sonderfall der Verbindungen der Formel (I): 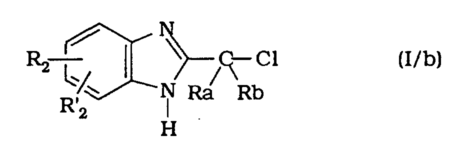 in der R₂, R'₂, Rₐ und R_{b} die bezüglich der Formel (I) angegebenen Bedeutungen
besitzen,
welche man klassischen Reaktionen, bei denen man Chloridderivate einsetzen kann, unterwerfen kann zur Bildung der entsprechend substituierten Verbindung,
welche Verbindungen der Formeln (I/a) und (I/b) man:
- bezüglich der NH-Funktion gegebenenfalls durch eine geradkettige oder verzweigte (C₁-C₆)-Alkylgruppe substituieren kann,
- gegebenenfalls mit Hilfe einer klassischen Reinigungsmethode reinigen kann,
- gegebenenfalls mit Hilfe einer klassischen Trennmethode in die Isomeren trennen kann und
- gewünschtenfalls in ihre Additionssalze mit einer pharmazeutisch annehmbaren Säure oder Base umwandeln kann.

17. Verfahren zur Herstellung der Verbindungen der Formel (I), dadurch gekennzeichnet, daß, wenn die herzustellenden Verbindungen der Formel (I) jene sind, worin X = X' = O oder S darstellt, das Verfahren dadurch gekennzeichnet ist, daß man als Ausgangsprodukt eine Verbindung der Formel (IV) verwendet: 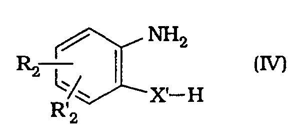 in der R₂ und R'₂ die bezüglich der Formel (I) angegebenen Bedeutungen besitzen und X' = O oder S bedeutet,
welche man in saurem Medium mit einer Verbindung der Formel (III) umsetzt: 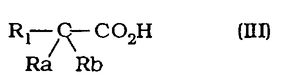 in der Rₐ, R_{b} und R₁ die bezüglich der Formel (I) angegebenen Bedeutungen besitzen,
zur Bildung der Verbindung der Formel (I/c), einem Sonderfall der Verbindungen der Formel (I): 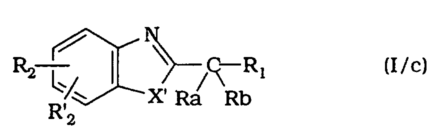 in der R₂, R'₂, X', Rₐ, R_{b} und R die oben angegebenen Bedeutungen besitzen,
welche man gegebenenfalls, wenn R₁ eine Hydroxygruppe darstellt, der Einwirkung von Thionylchlorid unterwirft,
zur Bildung der Verbindung der Formel (I/d), einem Sonderfall der Verbindungen der Formel (I): 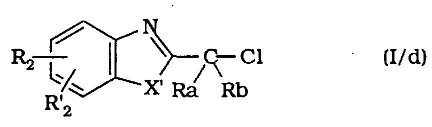 in der R₂, R'₂, Rₐ und R_{b} die oben angegebenen Bedeutungen besitzen,
welche anschließend klassischen Reaktionen unterworfen werden kann, bei denen man die chlorierten Derivate einsetzen kann zur Bildung der entsprechenden substituierten Verbindungen,
welche Verbindungen der Formeln (I/c) und (I/d) man:
- gegebenenfalls mit Hilfe einer klassischen Reinigungsmethode reinigen kann,
- gegebenenfalls mit Hilfe einer klassischen Trennmethode in ihre Isomeren auftrennen kann und
- gewünschtenfalls mit Hilfe einer pharmazeutisch annehmbaren Säure oder Base in ihre Additionssalze umwandeln kann.

18. Pharmazeutische Zubereitungen enthaltend als Wirkstoff mindestens eine Verbindung nach einem der Ansprüche 1 bis 15 alleinoder in Kombination mit einem oder mehreren inerten, nichttoxischen, pharmazeutisch annehmbaren Trägermaterialien oder Bindemitteln.

19. Pharmazeutische Zubereitungen nach Anspruch 18 enthaltend mindestens einen Wirkstoff nach einem der Ansprüche 1 bis 15 zur Verwendung als Inhibitoren von Interleukin 1β.

## Claims

1. Compounds of formula (I): 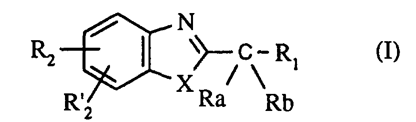 wherein:
R₁ represents a halogen atom, a hydroxy group, a linear or branched (C₁-C₆)alkoxy group (optionally substituted by an aryl group), a trihalomethyl group, an arylmethyl group, a cyano group, a sulpho group, an amino group (optionally substituted by one or more, identical or different, linear or branched (C₁-C₆)alkyl, optionally substituted aryl, acyl, arylsulphonyl or alkylsulphonyl groups), a mercapto group, a linear or branched (C₁-C₆)alkylthio group, a linear or branched (C₁-C₆)trialkylammonium group, an aryloxy group, an arylthio group, an arylaminocarbonyl group, an arylcarbonylamino group, an arylsulphonylamino group, an arylaminosulphonyl group, an arylureido group, an arylthioureido group, an arylsulphonyl group, an arylsulphonyloxy group, a (C₃-C₇)cycloalkyloxy group, a (C₃-C₇)cycloalkylthio group, a (C₆-C₈)bicycloalkyloxy group (optionally substituted by an aryl group) or a (C₆-C₈)bicycloalkylthio group (optionally substituted by an aryl group), it being understood that each aryl group may be optionally substituted,
Ra and Rb, which may be identical or different, represent a hydrogen atom, a hydroxy group or a linear or branched (C₁-C₆)alkyl group (optionally substituted by an aryl group),
X represents an oxygen or sulphur atom or a group NR (wherein R represents a hydrogen atom or a linear or branched (C₁-C₆)alkyl group),
R₂ represents an imidazolyl, benzimidazol-1-yl, naphthyl, pyridyl, thiazolyl, oxazolyl, benzothiazolyl, benzoxazolyl, pyrrolyl, furyl, thienyl, imidazopyridinyl, triazolyl or tetrazolyl group, each of those groups being optionally substituted by one or more, identical or different, halogen atoms, linear or branched (C₁-C₆)alkyl, linear or branched (C₁-C₆)trihaloalkyl, linear or branched (C₁-C₆)alkoxy, nitro, cyano, carboxy, linear or branched (C₁-C₆)alkoxycarbonyl, hydroxy, amino (optionally substituted by one or more, identical or different, linear or branched (C₁-C₆)alkyl, acyl, alkylsulphonyl or arylsulphonyl groups), optionally substituted phenyl or optionally substituted bicycloalkyl groups,
R'₂ represents a hydrogen atom or an optionally substituted aryl group,
their stereoisomers and addition salts thereof with a pharmaceutically acceptable acid or base.

2. Compounds of formula (I) according to claim 1 wherein X represents a group NR.

3. Compounds of formula (I) according to claim 1 wherein R₁ represents an optionally substituted aryloxy group.

4. Compounds of formula (I) according to claim 1 wherein R₁ represents an optionally substituted arylthio group.

5. Compounds of formula (I) according to claim 1 wherein R₁ represents an optionally substituted arylsulphonylamino group.

6. Compounds of formula (I) according to claim 2 wherein R₁ represents an optionally substituted phenoxy group.

7. Compounds of formula (I) according to claim 4 wherein R₁ represents an optionally substituted phenylthio group.

8. Compounds of formula (I) according to claim 5 wherein R₁ represents an optionally substituted phenylsulphonylamino group.

9. Compounds of formula (I) according to claim 1 wherein R₂ represents an optionally substituted imidazolyl group.

10. Compound of formula (I) according to claim 1 which is 2-phenoxymethyl-5-(imidazol-1-yl)benzimidazole, and addition salts thereof.

11. Compound of formula (I) according to claim 1 which is 2-phenylthiomethyl-5-(imidazol-1-yl)benzimidazole, and addition salts thereof.

12. Compound of formula (I) according to claim 1 which is 2-(2,6-dimethylphenylthiomethyl)-5-(imidazol-1-yl)benzimidazole dihydrochloride.

13. Compound of formula (I) according to claim 1 which is 2-(2,6-dimethoxyphenoxymethyl)-5-(imidazol-1-yl)benzimidazole dihydrochloride.

14. Compound of formula (I) according to claim 1 which is 2-(phenylsulphonylaminomethyl)-5-(imidazol-1-yl)benzimidazole dihydrochloride.

15. Compound of formula (I) according to claim 1 which is 2-(2,6-dimethoxyphenoxymethyl)-5-(imidazol-1-yl)-6-(4-fluorophenyl)benzimidazole.

16. Process for the preparation of compounds of formula (I), characterised in that, when the desired compounds of formula (I) are those wherein X = NR, the process is characterised in that there is used as starting material a compound of formula (II) : 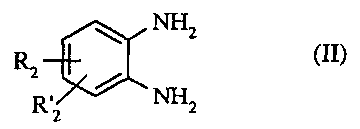 wherein R₂ and R'₂ are as defined for formula (I),
which is reacted, in an acidic medium, with a compound of formula (III) : 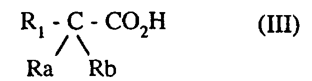 wherein Rₐ, R_{b} and R₁ have the same meanings as for formula (I),
to yield a compound of formula (I/a), which is a particular case of the compounds of formula (I): 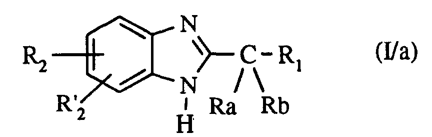 wherein R₁, Rₐ, R_{b}, R₂ and R'₂ are as defined for formula (I),
which, when R₁ represents a hydroxy group, is optionally subjected to the action of thionyl chloride,
to yield a compound of formula (I/b), which is a particular case of the compounds of formula (I): 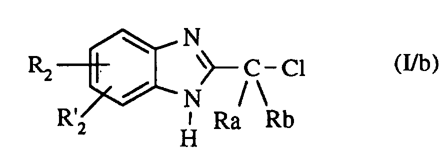 wherein R₂, R'₂, Rₐ and R_{b} have the same meanings as for formula (I),
which may then be subjected to the conventional reactions that can be carried out on chlorinated compounds, to obtain the corresponding substitutions,
which compound of formula (I/a) or (I/b) :
- the NH function of which may be optionally substituted by a linear or branched (C₁-C₆)alkyl group,
- may, if necessary, be purified in accordance with a conventional purification technique,
- is separated, where appropriate, into its isomers in accordance with a conventional separation technique,
- is converted, if desired, into addition salts thereof with a pharmaceutically acceptable acid or base.

17. Process for the preparation of compounds of formula (I) characterised in that, when the desired compounds of formula (I) are those wherein X= X' = O or S, the process is characterised in that there is used as starting material a compound of formula (IV) : 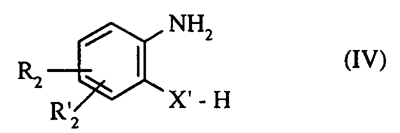 wherein R₂ and R'₂ are as defined for formula (I) and X' = O or S,
which is reacted, in an acidic medium, with a compound of formula (III) : 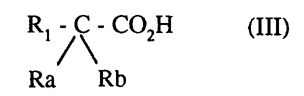 wherein Rₐ, R_{b} and R₁ have the same meanings as for formula (I),
to yield a compound of formula (I/c), which is a particular case of the compounds of formula (I): 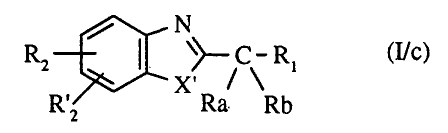 wherein R₂, R'₂, X', Rₐ, R_{b} and R are as defined hereinabove, which, when R₁ represents a hydroxy group, is optionally subjected to the action of thionyl chloride,
to yield a compound of formula (I/d), which is a particular case of the compounds of formula (I) : 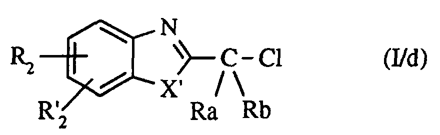 wherein R₂, R'₂, Rₐ and R_{b} have the same meanings as hereinabove,
which may then be subjected to the conventional reactions that can be carried out on chlorinated compounds, to yield the corresponding substitutions,
which compound of formula (I/c) or (I/d) :
- may, if necessary, be purified in accordance with a conventional purification technique,
- is separated, where appropriate, into its isomers in accordance with a conventional separation technique,
- is converted, if desired, into addition salts thereof with a pharmaceutically acceptable acid or base.

18. Pharmaceutical compositions comprising as active ingredient at least one compound according to any one of claims 1 to 15 on its own or in combination with one or more inert, nontoxic pharmaceutically acceptable excipients or carriers.

19. Pharmaceutical compositions according to claim 18 comprising at least one active ingredient according to any one of claims 1 to 15 for use as inhibitors of interleukin 1β.
